# NEUE EUROPÄISCHE PATENTSCHRIFT

(11) **EP 0 650 053 B2**
(45) Veröffentlichungstag und Bekanntmachung des Hinweises auf die Entscheidung über den Einspruch: **30.11.2005**
(45) Hinweis auf die Patenterteilung: 12.12.2001
(21) Anmeldenummer: 94116387.5
(22) Anmeldetag: 17.10.1994
(51) Int. Cl.: G01N 33/53, G01N 33/68

(54) **Synthetischer Standard für Immunoassays**
Synthetic standard for immunoassays
Standard synthétique pour essais immunologiques

(30) Priorität: 20.10.1993 DE 4335798; 20.05.1994 DE 4417735; 15.06.1994 DE 4420742
(43) Veröffentlichungstag der Anmeldung: 26.04.1995
(73) Patentinhaber: Roche Diagnostics GmbH, 68305 Mannheim (DE)
(72) Erfinder: Seidel, Christoph, Dr., D-82362 Weilheim (DE); Bialk, Peter, Dr., D-82390 Eberfing (DE); von der Eltz, Herbert, Dr., D-82362 Weilheim (DE)

(56) Entgegenhaltungen:
- EP-A- 0 306 813
- EP-A- 0 312 173
- EP-A- 0 339 695
- EP-A- 0 394 819
- EP-A- 0 472 205
- FEBS Letters vol. 328, no.1,2, p.193-144. 1993. Mesnard et al.

### SEQUENZPROTOKOLL

(1) ALGEMEINE INFORMATION:
   (i) ANMELDER:
      (A) NAME: Boehringer Mannheim GmbH
      (B) STRASSE: Sandhoferstrasse 116
      (C) ORT: Mannheim
      (E) LAND: DE
      (F) POSTLETTZAHL: 68298
   (ii) ANMELDETTTEL: Synthetischer Standard fuer Immunoassays
   (iii) ANZAHL DER SEQUENZEN: 11
   (iv) COMPUTER-LESBARE FORM:
      (A) DATENTRÄGER: Tape
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 11 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 1:
   Leu Lys Asp Arg Ile Glu Lys Arg Arg Ala Glu
   1               5                   10
(2) INFORMATION ZU SEQ ID NO: 2:
   (i) SEQUENZ CHARAKTERISTIKA:
      (A) LÄNGE: 8 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 2:
   Leu Ile Glu Ala His Phe Glu Asn
   1               5
(2) INFORMATION ZU SEQ ID NO: 3:
   (i) SEQUENZ CHARAKTERISTIKA:
      (A) LÄNGE: 11 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 3:
   Leu Gln Ala Leu Ile Glu Ala His Phe Glu Asn
   1               5                   10
(2) INFORMATION ZU SEQ ID NO: 4:
   (i) SEQUENZ CHARAKTERISTIKA:
      (A) LÄNGE: 14 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 4:
   Leu Asn Glu Leu Gln Ala Leu Ile Glu Ala His Phe Glu Asn
   1 5 10
(2) INFORMATION ZU SEQ ID NO: 5:
   (i) SEQUENZ CHARAKTERISTIKA:
      (A) LÄNGE: 9 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 5:
   Pro Pro Lys Ile Pro Asp Gly Glu Arg
   1 5
(2) INFORMATION ZU SEQ ID NO: 6:
   (i) SEQUENZ CHARAKTERISTIKA:
      (A) LÄNGE: 12 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 6:
   Glu Gln Gln Arg Ile Arg Asn Glu Arg Glu Lys Glu
   1 5 10
(2) INFORMATION ZU SEQ ID NO: 7:
   (i) SEQUENZ CHARAKTERISTIKA:
      (A) LÄNGE: 8 Aminosäuren
      (B) ART: Aminosäure
      (C) STRANGFORM: Einzel
      (D) TOPOLOGIE: linear
   (ii) ART DES MOLEKULS: Peptid
   (xi) SEQUENZBESCHREIBUNG: SEQ ID NO: 7:
   Ala Leu Ser Asn Met Met His Phe
   1 5
(2) INFORMATION ZU SEQ ID NO: 8:
   (i) SEQUENZ CHARAKTERISTIKA:
      (A) LÄNGE: 9 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 8:
   Met Pro Asn Leu Val Pro Pro Lys Ile
   1 5
(2) INFORMATION ZU SEQ ID NO: 9:
   (i) SEQUENZ CHARAKTERISTTKA:
      (A) LÄNGE: 10 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 9:
   Leu Gln Glu Lys Phe Lys Gln Gln Lys Tyr
   1 5 10
(2) INFORMATION ZU SEQ ID NO: 10:
   (i) SEQUENZ CHARAKTERISTIKA:
      (A) LÄNGE: 10 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 10:
   Val Leu Arg Asn Arg Ile Asn Asp Asn Gln
   1               5                   10
(2) INFORMATION ZU SEQ ID NO: 11:
   (i) SEQUENZ CHARAKTERISTIKA:
      (A) LÄNGE: 7 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 11:
   Gly Pro Arg Val Val Glu Arg
   1               5

## Beschreibung

Gegenstand der Erfindung ist ein peptidischer synthetischer Standard, der in Immunoassays verwendet werden kann.

Die in der Immunologie zu bestimmenden Analyten sind oft Humanproteine, wie z. B. Creatinkinase (z. B. CKMB), HCG, Fibrin Monomer, Prolactin oder Troponin T. Zur Kalibrierung von derartigen Tests werden üblicherweise aus natürlichen Quellen isolierte Humanproteine verwendet. Diese Isolierung ist schwierig, kostenaufwendig und bedingt den Umgang mit gegebenenfalls infektiösem Humanmaterial. Dabei muß auch sichergestellt werden, daß zur Kalibrierung verwendete Humanproteine keine infektiösen Verunreinigungen enthalten. Außerdem sind die Humanproteine nicht unter allen Bedingungen stabil. Beispielsweise zeigt Troponin T in Lösung keine ausreichende Langzeitstabilität wie sie für einen Flüssigstandard erforderlich wäre. Außerdem ist es praktisch unmöglich, einen stabilen gemeinsamen Standard von solchen Humanproteinen bei gleichen Pufferbedingungen zu formulieren, da die Stabilitätsmaxima der Proteine meist in unterschiedlichen und dazu noch sehr engen pH-Bereichen zu finden sind.

Das Ziel der Erfindung ist es, für immunologische Bestimmungsverfahren zum Nachweis von Humanproteinen über Bindung des Analyten an mindestens einen analytspezifischen Rezeptor Standards bereitzustellen, die stabil sind und einfach und preiswert herzustellen sind und die oben beschriebenen Nachteile nicht besitzen.

Gelöstwird diese Aufgabe durch einen flüssig stabilen Kalibrator zur Verwendung in einem Test zum Nachweis eines Analyten, bestehend aus einem Konjugat aus mindestens zwei Bindungsstellen, die spezifisch mit der analytspezifischen Binderegion des Rezeptors, der zum Nachweis im Test eingesetzt wird, binden, wobei die Bindungsstellen durch mindestens eine lösliche Trägersubstanz verbunden sind und das Konjugat das Produkt einer synthetischen oder rekombinanten Herstellung ist, gelöst in einer wässrigen Lösung in einer genau bekannten Menge.

Ein weiterer Gegenstand der Erfindung ist die Verwendung dieses flüssig stabilen Kalibrators zur Erstellung einer Eichkurve in einem Test zum Nachweis eines Analyten.

Ein weiterer Gegenstand der Erfindung ist ferner ein Verfahren zum Nachweis eines Analyten in einer Probe durch Bindung des Analyten an mindestens einen analytspezifischen Rezeptor und Nachweis des gebildeten Komplexes als Maß für den Gehalt an Analyt in der Probe, welches dadurch gekennzeichnet ist, daß
a) in einem ersten Schritt eine bekannte Menge eines erfindungsgemäßen Konjugats anstelle der Probe mit dem analytspezifischen Rezeptor gebunden wird,
b) der gebildete Komplex zwischen Konjugat und Rezeptor bestimmt wird,
c) das Meßergebnis aus Schritt b) zur Erstellung einer Eichkurve verwendet wird,
d) der Nachweis des Analyten in der Probe durchgeführt wird und die Analytkonzentration durch Bezug des Meßsignals auf die aus c) erhaltene Eichkurve ermittelt wird.

Unter einer Eichkurve ist die Zuordnung von einer Mehrzahl von Meßwerten zu Analytkonzentrationen zu verstehen.

Es hat sich überraschenderweise gezeigt, daß ein solches Konjugat in Immunoassays als synthetischer Standard geeignet und in Lösung in weiten pH-Bereichen langzeitstabil ist. Konjugate aus jeweils nur einer Bindungsstelle, die spezifisch mit der analytspezifischen Binderegion des Rezeptors, der zum Nachweis im Test eingesetzt wird, bindet, sind als Standards in solchen Tests weniger geeignet und besitzen nur eine geringe Affinität zu den Rezeptoren.

Unter Rezeptoren werden alle üblichen, dem Fachmann bekannten Moleküle verstanden, die mit dem nachzuweisenden Analyten binden, also beispielsweise Antikörper oder Bindeproteine. Besonders bevorzugt werden solche Rezeptoren eingesetzt, die mindestens zwei analytspezifische Binderegionen aufweisen, also beispielsweise Antikörper oder deren bivalente Fragmente (F(ab)₂). Das Vorliegen von zwei analytspezifischen Binderegionen im Rezeptor ermöglicht die zweiarmige Bindung zum erfindungsgemäßen Kalibrator, in dem mindestens zwei Bindungsstellen pro Konjugat für den Rezeptor vorhanden sind, wodurch im Vergleich zu einem Konjugat, das lediglich eine Bindungsstelle besitzt, die Bindekonstante durch die Avidität des Rezeptors zum Konjugat um das 1.000- bis 10.000-fache gesteigert wird.

Auch für monoklonale Rezeptoren wie Fab'-Fragmente ist die Verwendung der erfindungsgemäßen Kalibratoren vorteilhaft.

Diese multivalenten Verbindungen ermöglichen die Bindung einer größeren Zahl von gelabelten Rezeptoren, was zu einem deutlich erhöhten Signal führt. Festphasengebundene monovalente Rezeptoren sind in ihen Eigenschaften vergleichbar mit festphasengebundenen multivalenten Rezeptoren, so daß die erfindungsgemäßen Peptidderivate auch vorteilhaft für monovalente festphasengebundene Rezeptoren sind.

Unter einer Bindungsstelle ist ein Peptid zu verstehen, dessen Sequenz einen Teil der Proteinsequenz eines Proteinantigens (Analyt) ist und an das ein gegen dieses Protein gerichteter Rezeptor spezifisch bindet. Neben diesen Peptiden sind unter einer Bindungsstelle auch noch Peptide mit Aminosäuresequenzen zu verstehen, die eine im wesentlichen äquivalente Spezifität und/oder Affinität der Bindung an den Rezeptor, wie die zuvor genannten Peptide zeigen. Diese Peptide können vorzugsweise durch Substitution, Deletion oder Insertion einzelner Aminosäurereste aus den zuvor genannten Peptiden abgeleitet werden.

Unter einer Bindungsstelle sind auch Peptid-Derivate der zuvor genannten Peptide zu verstehen, in denen eine oder mehrere Aminosäuren durch eine chemische Reaktion derivatisiert worden sind. Beispiele von erfindungsgemäßen Peptidderivaten sind insbesondere solche Moleküle, in denen das Backbone oder/und reaktive Aminosäureseitengruppen, zum Beispiel freie Aminogruppen, freie Carboxylgruppen oder/und freie Hydroxylgruppen, derivatisiert worden sind. Spezifische Beispiele für Derivate von Aminogruppen sind Sulfonsäure- oder Carbonsäureamide, Thiourethanderivate und Ammouniumsalze, zum Beispiel Hydrochloride. Beispiele für Carboxylgruppenderivate sind Salze, Ester und Amide. Beispiele für Hydroxylgruppenderivate sind 0-Acyl- oder 0-Alklyderivate.

Weiterhin umfaßt der Begriff Peptidderivat auch solche Peptide, in denen eine oder mehrere Aminosäuren durch natürlich vorkommende oder nicht-natürlich vorkommende Aminosäurehomologe der 20 "Standard"-Aminosäuren ersetzt werden. Beispiele für solche Homologe sind 4-Hydroxyprolin, 5-Hydroxylysin, 3-Methylhistidin, Homoserin, Ornithin, β-Alanin und 4-Aminobuttersäure. Die Peptidderivate müssen eine im wesentlichen äquivalente Spezifität oder/und Affinität der Bindung an die Rezeptoren aufweisen, wie die Peptide, aus denen sie abgeleitet sind.

Unter einer Bindungsstelle sind auch peptidmimetische Substanzen, im folgenden Peptidmimetika genannt, zu verstehen, die eine im wesentlichen äquivalente Spezifität oder/und Affinität der Bindung an die Rezeptoren aufweisen, wie die zuvor genannten Peptide oder Peptidderivate. Peptidmimetika sind Verbindungen, die Peptide in ihrer Wechselwirkung mit dem Rezeptor ersetzen können und gegenüber den nativen Peptiden eine erhöhte Stabilität, insbesondere gegenüber Proteinasen oder Peptidasen, aufweisen können. Methoden zur Herstellung von Peptidmimetika sind beschrieben bei Giannis und Kolter, Angew. Chem. 105 (1993), 1303 - 1326 und Lee et al., Bull. Chem. Soc. Jpn. 66 (1993), 2006 - 2010.

Die Länge einer Rezeptorbindungsstelle beträgt üblicherweise mindestens 4 Aminosäuren. Bevorzugt liegt die Länge zwischen 4 bis 20, 4 bis 15 oder besonders bevorzugt 4 bis 10 Aminosäuren. Im Falle der Peptidmimetika ist eine analoge Länge beziehungsweise Größe des Moleküls notwendig.

Für die Bestimmung von Troponin T sind beispielsweise als Bindungsstellen folgende Peptide geeignet:

Besonders geeignet sind Peptide gemäß SEQ ID NO. 1, 2, 3, 4 und/oder SEQ ID NO. 6. Die gesamte DNA und Aminosäuresequenz von Troponin T ist beschrieben in Mesnard, L. et al FEBS Letters 328, 1.2, (1993) 139 - 144.

Für die Bestimmung von Fibrin-Monomer sind beispielsweise als Bindungsstellen folgende Peptide geeignet:

Besonders geeignet sind die erfindungsgemäßen Konjugate als Standards zur Bestimmung von Troponin T, HCG und CKMB und Fibrinmonomer in immunologischen Sandwich-Tests. Immunologische Sandwich-Tests für HCG, CKMB und Fibrinmonomer sind beispielsweise beschrieben in:
J. F. O'Connor
   Cancer Research 48, 1361 - 1366, (1988)
S. K. Gupta
   Journal of immunological Methods 83 (1985) 159 - 168
B. Longhi
   Journal of immunological Methods 92 (1986) 89 - 95
T. Chard
   Human Reproduction vol 7 no 5 pp 701 - 710 (1992)
L. R. Witherspoon
   Clin. Chem. 38/6 887 - 897 (1992)
Clin. Chem. 38/1, 144 - 147 (1992)
R. J. Norman
   Ann. Clin. Biochem. (1990) 27:183 - 194
H. Christensen
   Clin. Chem. 36/9, 1686 - 1688 (1990)
S.D. Figard
   Eur. J. Clin. Chem. Clin. Biochem., 28, 1991, 77 - 80
U. Scheefers-Borchel
   Proc. Natl. Acad. Sci. USA 82 (1985) 7091 - 7095
H. Lill
   Blood Coag. Fibrinol. 4 (1993) 97 - 102
C. E. Dempfle
   Blood Coag. Fibrinol. 4 (1993) 79 - 86
Zabel, M.
   Circulation (1993) 87 (5) p1542 - 50
Van Blerk, M.
   Clin. Chem. 38, 12 (1992) pp2380 - 6
Buttery, J. E.
   Clin. Biochem. 25,1 (1992) 11 - 13

Ein Verfahren zur Bestimmung von Troponin T ist beschrieben in EP-A-0 394 819.

Die Auswahl von Bindungsstellen für beliebige Proteinanalyten ist nach Verfahren möglich, die jedem Fachmann geläufig sind. Solche Verfahren sind beispielsweise beschrieben in V. Regenmortel, Synthetic Polypeptides as Antigens, Elsevier 1988, 1 -16.

Das Konjugat, das als synthetischer Standard in Immunoassays geeignet ist, enthält mindestens je zwei gleiche Bindungsstellen, die spezifisch mit der analytspezifischen Binderegion zweier unterschiedlicher Rezeptoren, die zum Nachweis im Test eingesetzt werden, binden. Im Falle eines Assays, bei dem zwei analytspezifische Rezeptoren notwendig sind, die beide gleichzeitig mit dem Analyten zu binden vermögen, beispielsweise Sandwich-Immunoassays, enthält das Konjugat jeweils zwei gleiche oder ähnliche Bindungsstellen, die mit den zwei unterschiedlichen Rezeptoren zu binden vermögen. Bei diesen Assays werden zwei unterschiedliche Rezeptoren verwendet, die nicht um das gleiche Epitop des Analyten konkurrieren. In diesem Fall enthält das Konjugat also zwei Bindungsstellen für den ersten und zwei Bindungsstellen für den zweiten Rezeptor.

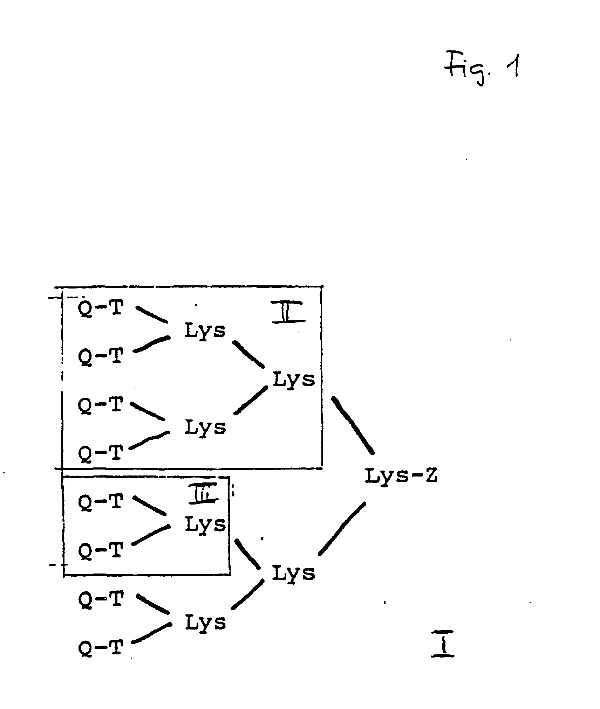

Die Anzahl der Bindungsstellen kann aber auch größer sein. Enthält das Konjugat ein lösliches Polymeres von hohem Molekulargewicht als Trägersubstanz, können bis zu 50 Bindungsstellen je Rezeptor-Binderegion enthalten sein. Wenn in dem Konjugat die Bindungsstellen mit der Trägersubstanz zu einem linearen oder verzweigten synthetischen Oligomeren aus Aminosäuren und Spacermolekülen gekoppelt ist, wird als Anzahl der Bindungsstellen 2 bis 16 bevorzugt. In linearen Konjugaten besteht die Trägersubstanz bevorzugt aus einer Mehrzahl von peptidischen Oligomeren, die zwischen den Bindungsstellen angeordnet sind. Die Trägersubstanz ist in solchen Fällen vorzugsweise identisch mit dem Spacer. Verzweigte Konjugate besitzen vorzugsweise eine Baumstruktur (vgl. z. B. Fig. 1). In solchen Konjugaten beträgt die Anzahl der Bindungsstellen vorzugsweise 2, 4, 8 oder 16. Hierbei ist es bevorzugt, daß zwischen den Enden der Trägersubstanz und den Bindungsstellen Spacer eingefügt sind.

In einer bevorzugten Ausführungsform des Konjugats sind die Bindungsstellen über Verzveigungestellen mit 2, 4 oder 8 Aminofunktionen als Trägersubstanz miteinander kovalent verbunden. Bei einer solchen Verzweigungsstelle handelt es sich um ein Di- oder mehrfachfunktionelles Molekül, an dessen Funktionalitäten Bindungsstellen und ggf. auch weitere Verzweigungsstellen gebunden sein können. Diese Bindung kann direkt oder über einen Spacer erfolgen. Bevorzugte Verzweigungsstellen sind beispielsweise α-Bisaminosäuren, wie Lysin oder α-Bisamine. Derartige verzweigte Peptide sind beispielsweise beschrieben in Tam, J.P. Proc. Natl. Acad. Sci. USA 85 (1988) 5409 - 5431, WO 92/18528 und Marsden, H.S. et al., J. Immunol. Meth. 147 (1992) 65 - 72.

In EPA 339 695 wind die Herstellung von antigenen oder immunogenen Konjugaten, die als "multiple antigene peptides" (MAPs) bezeichnet werden offenbart und beschrieben, dass diese MAPs in pharmazeutisch wirksamen Produkten, z.B. als Antigene, Immunogene oder Vakzine eingesetzt werden können.

Unter der Funktionalität einer Verzweigungsstelle versteht man eine chemische Gruppe, die an eine chemische Gruppe in einer Bindungsstelle (gegebenenfalls nach vorheriger Aktivierung) binden kann. Beispiele hierfür sind primäre oder sekundäre Amin-, Carbonsäure-, Sulfhydryl-, Alkohol-, Aldehyd- und Sulfonsäurefunktionen.

Ebenfalls bevorzugt als Trägersubstanz für Konjugate mit Baumstruktur sind Lysin und Homooligomere von Lysin, vorzugsweise mit 3 bis 7 Lysinen.

Die Bindungsstellen, die jeweils gegen den gleichen Rezeptor gerichtet sind, können entweder in ihrer Aminosäuresequenz gleich oder verschieden sein. Es muß jedoch gewährleistet sein, daß die Bindungsstellen dieselbe Binderegion der Rezeptoren spezifisch erkennen.

Die Bindungsstellen sind zweckmäßig über Spacer mit 3 - 40 Atomen Länge miteinander und/oder mit der Trägersubstanz kovalent zum Konjugat verbunden. Unter einem Spacer versteht man ein bifunktionelles Molekül, das kovalent an die Enden zweier Bindungsstellen bzw. an eine Bindungsstelle und an den löslichen Träger binden kann und dadurch zwischen diesen Bindungsstellen eine räumliche Distanz erzeugt.

Die Spacer sind vorzugsweise lineare Moleküle mit für die Wasserlöslichkeit ausreichend polaren Eigenschaften. Besonders bevorzugt besteht auch der Spacer aus Aminosäureresten. Diese Aminosäuren können der natürlichen Zwischensequenz der Bindungsstellen im natürlichen Analyten entsprechen oder von der natürlichen Sequenz abweichend sein. Hierbei sind wiederum natürliche Aminosäuren, wie beispielsweise Alanin, Glycin, Cystein, Lysin, Asparginsäure, Glutaminsäure, Serin, Leucin oder nicht-natürliche lineare ω-Aminosäuren wie z.B. β-Alanin, γ-Aminobuttersäure oder ω-Aminocapronsäure bevorzugt. Ihre Kopplung erfolgt über kovalente Bindungen (z. B. -CO-NH-, -NH-CO-, -S-S-, -CO-O-, -O-CO-) oder über bivalente Spacer. Die Spacerlänge muß so gewählt werden, daß die Rezeptoren weitgehend ungehindert an die Bindungsstellen binden können. Eine Spacerlänge über 40 Atome zeigt keine besonderen Vorteile mehr. Außerdem sind derartige Moleküle synthetisch aufwendiger herzustellen.

Vorzugsweise bestehen die Spacer aus jeweils 1 - 10 Aminosäuren. Bevorzugte Spacer sind: Oligoglycine, Oligo-β-Alanine, Oligo-ω-Aminocapronsäuren oder Oligopeptide aus β-Alaminen und ω-Aminocapronsäuren. Der N-Terminus des Konjugats kann entweder frei sein oder einen organischen Rest gebunden enthalten, wie z. B. den Acetyl-, Tertiärbutyloxycarboxyl-, Peptidyl-, Aryl- Alkylrest. Der C-Terminus kann ebenfalls frei vorliegen als Carboxylat oder Amid, oder er kann mit einem organischen Rest derivatisiert sein und beispielsweise ein Alkylester oder Arylester sein.

In einer bevorzugten Ausführungsform sind C-Terminus und N-Terminus über einen bivalenten Spacer oder eine Peptidsequenz verbunden, so daß das Molekül eine cyklische Struktur aufweist.

In einerweiteren Ausführungsform kann dieTrägersubstanz eine Kombination aus Spacer und Verzweigungsstellen sein und ein Polymer darstellen, welches eine Mehrzahl von funktionellen Bindungsgruppen für die Bindungsstellen besitzt. Derartige Polymere können beispielsweise lösliche Polyamine, Dextrane, Polyacrylate, Polymethacrylate, Polypeptide, Proteine oder Nucleinsäuren sein mit einem Molekulargewicht von 1000 bis 5 Mio. Dalton, bevorzugt aber von 10000 bis 1 Mio. Dalton. Als Polymere sind insbesondere lösliche Proteine wie z.B. Albumine, Immunglobuline oder β-Galactosidase geeignet. Die Polymere tragen 2 bis 500 kopplungsfähige Gruppen. Dem Fachmann sind eine große Zahl von geeigneten Methoden für die Herstellung der Bindung zwischen Bindestellen und Polymeren bekannt. Zur Bindung der Bindungsstellen an die Polymere werden die Polymere zweckmäßig aktiviert. Die Bindung kann z.B. über hetero- oder homobifunktionelle Linker oder über die direkte Ausbildung einer Amidbindung erfolgen. Besonders zweckmäßig ist die Umsetzung eines Aminogruppen tragenden Polymers mit dem N-Hydroxysuccinimidesterderivat der Bindestelle. Besonders geeignet ist auch die direkte Umsetzung eines SH-Gruppen tragenden Polymers mit Maleinimidderivaten der Bindestelle, bzw. die Verwendung von maleinimid-derivatisierten Trägerpolymeren und SH-Gruppen tragende Bindestellen.

Die erfindungsgemäßen verwendeten Konjugate können nach bekannten Methoden hergestellt werden, wie sie beispielsweise bei J.P. Tam, Proc. Natl. Acad. Sci. USA, 58 (1988), 5409 - 5413; D.N. Pressmett et al., J. Biol. Chem. 263 (1988); 1719 - 1725; H. Lankinen, J. Gen. Virol. 70 (1989), 3159 - 3169; WO 92/18528 oder H.S. Marsden, J. Immunol. Methods 147 (1992), 65 - 72 beschrieben sind.

Die erfindungsgemäßen Konjugate aus mindestens zwei Bindungsstellen und mindestens einer löslichen Trägersubstanz werden als flüssige Kalibratoren zur Erstellung einer Eichkurve in einem Test zum Nachweis eines Analyten verwendet. Bei der Bereitstellung der erfindungsgemäßen flüssigen Kalibratoren ist darauf zu achten, daß die Konjugate in einer genau definierten und bekannten Menge im flüssigen Kalibrator vorliegen. Die flüssigen Kalibratoren werden zweckmäßigerweise zur Erstellung einer Eichkurve in verschiedenen Konzentrationen angeboten, damit keine weiteren Verdünnungsschritte vor der Benutzung notwendig sind. Die Konzentration des Konjugats im flüssigen Kalibrator richtet sich nach dem nachzuweisenden Analyten und deckt üblicherweise die physiologisch auftretenden Analytkonzentrationen ab. Kalibrator-Reihen werden in typischen Konzentrationen von 0,1 bis 1000 ng/ml angeboten. Eine flüssige Kalibrator-Reihe für den Troponin T-Test deckt beispielsweise die Konjugatkonzentrationen von 0; 0,1; 0,5; 1; 5; 10; 50; 100 und 500 ng/ml ab. Beim Fibrin-monomer-Test beträgt die Konjugat-Konzentration in der Kalibrator-Reihe beispielsweise, 10, 50, 100, 250 und 500 ng/ml.

Die Bezeichnung flüssig stabiler Kalibrator bedeutet, daß nach Lösung einer genau definierten Menge des Konjugates in einer bestimmten Menge eines Lösungsmittels, üblicherweise eines Puffers, der dadurch entstandene flüssige Kalibrator lang stabil ist. Das Lösen des Konjugates im Lösungsmittel kann bereits beim Hersteller erfolgen. Dies hat den Vorteil, daß der Endverbraucher keine zusätzlichen Arbeitsschritte durchzuführen braucht. Andererseits kann das Konjugat auch vom Anwender aufgelöst werden. Das Konjugat wird als trockene Substanz, beispielsweise als Lyophilisat, vom Hersteller bereitgestellt. Dies hat den Vorteil, daß beispielsweise ein unsachgemäßer Transport oder eine unsachgemäße Lagerung vor der Benutzung, das heißt Auflösung, weniger schädlich ist.

Der flüssige Kalibrator enthält neben dem Konjugat in einer genau definierten und bekannten Menge noch weitere Hilfsstoffe wie beispielsweise Puffer, Stabilisatoren oder Konvervierungsmittel. Als Puffer haben sich Natriumphosphat, MES oder PIPES als besonders geeignet erwiesen. Der pH-Wert beträgt vorzugsweise 5,0 - 6,0. Als Stabilisatoren kann beispielsweise Rinder-serumalbumin eingesetzt werden. Die erfindungsgemäßen Stabilisatoren weisen eine höhere Stabilität bei der Lagerung auf als Stabilisatoren, die aus den natürlich vorkommenden Analyten hergestellt wurden. Besonders deutlich wird dieser Effekt, wenn es sich bei den Analyten um ein labiles Protein handelt wie beispielsweise Troponin T. Ursachen können zum Beispiel sein proteolytischen Angriff, unspezifische Bindung an Oberflächen oder Aggregationsneigung.

Ein weiterer Gegenstand der Erfindung ist ein flüssig stabiler Universalkalibrator zur Verwendung in mehreren Tests zum Nachweis von mindestens zwei unterschiedlichen Analyten.

Der Universalkalibrator besteht aus einer Mischung von mindestens zwei unterschiedlichen der zuvor beschriebenen Kalibratoren. Da es sich bei diesen synthetischen Kalibratoren um genau definierte Substanzen handelt, ist die gegenseitige Beeinflussung im Gemisch sehr geringer als bei Material aus organischer Quelle.

Verfahren zum Nachweis eines Analyten in einer Probe durch Binden des Analyten an mindestens einen analytspezifischen Rezeptor sind dem Fachmann geläufig und beispielsweise in D.W. Chang, M.T. Perlstein, Immunoassay-a practical guide, Academic Press, Inc. (1987), Wisdom, Clin. Chem. 22/8 (1976), 1243 - 1255, Oellerich, J. Clin. Chem. Clin. Biochem 18 (1980), 197 - 208 und Kage und Kottgen, mta 3 (1988), 10, 797 - 804 beschrieben.

Die erfindungsgemäßen Konjugate können sowohl in homogenen Immunoassays als auch in heterogenen Immunoassays verwendet werden. Unter homogenen Assays werden beispielsweise die Präzipitations-Methode, beispielsweise TINIA, die Agglutinations-Methode, FPIA (Fluoreszenzpolerisations-Immunoassay) oder CEDIA, also Methoden, bei denen das Maß der Rezeptor-Analyt-Reaktion ohne Trennung der freien und gebundenen Teile quantifiziert wird, verstanden. Unter heterogenen Assays werden beispielsweise Radioimmunoassays oder Sandwich-Assays, also Methoden, bei denen das Maß der Rezeptor-Analyt-Reaktion nach Trennung der freien von gebundenen Teilen quantifiziert wird, verstanden. Bei Sandwich-Immunoassays werden als Markierung beispielsweise enzymatische Markierung oder Fluoreszenzmarkierungen verwendet. Derartige Markierungen sind dem Fachmann bekannt und beispielsweise beschrieben in:
D. L. Bates
   Trends in Biotechnology 5 (7) (1987) pp204 - 209
M. A. Ator
   JBC 262, 31, 14954 - 14960 (1987)
K. Beyzavi
   Ann. Clin. Biochem. 24, 145 - 152 (1987)
J. Ennen
   mta 4 (1989) 3, S. 199 - 203
J. Ennen
   mta 4 (1989) 5, S. 414 - 416
Ekins, R.
   J. Biolumin. Chemilumin., July 1989, 4 (1), p59 - 78
Helgert K.
   Pharmazie Nov. 1989, 44 (11) p745 - 52
Ishikawa, E.
   J. Clin. Lab. Anal. 1989, 3 (4) p252 - 65
Porstmann,
   J. Immunol. Methods, June 24, 1992, 150 (1-2) p5 - 21
Ishikawa, E.
   Mol. Cell Probes, April 1991, 5 (2) p 81 - 95
Weber, T. H.
   Scand. J. Clin. Lab. Invest. Suppl., 1990, 201, p77 - 82
Walker, M. R.
   Methods Biochem. Anal. 1992, 36, p179 - 208
King, W. J.
   Immunol. Ser. 1990, 53, p 83 - 93
Ishikawa, E.
   Clin. Chim. Acta, Dec. 17, 1990, 194 (1), p51 - 72

Als besonders geeignet haben sich die erfindungsgemäßen Konjugate zur Verwendung bei Sandwich-Assays erwiesen. Die Bestimmung erfolgt dabei in der Weise, daß die Probe mit mindestens zwei analytspezifischen Rezeptoren inkubiert wird, wobei einer dieser Rezeptoren vor oder nach der Reaktion mit dem Analyten in eine Festphase immobilisiert wird und der zweite Rezeptor eine Markierung trägt. Nach beendeter immunologischer Reaktion werden flüssige Phase und Festphase getrennt und die Markierung in einer der beiden Phasen als Maß für die Menge an Analyt bestimmt.

Bei dem Verfahren zum Nachweis eines Analyten in einer Probe durch Bindung des Analyten an mindestens einen analytspezifischen Rezeptor und Nachweis des gebildeten Komplexes als Maß für den Gehalt des Analyten in der Probe wird in einem ersten Schritt anstelle der Probe eine bekannte Menge des erfindungsgemäßen Konjugats mit dem analytspezifischen Rezeptor gebunden. Nach der Bestimmung des gebildeten Komplexes zwischen Konjugat und Rezeptor wird aus diesen Ergebnissen eine Eichkurve erstellt. Danach wird der Nachweis des Analyten durchgeführt und die Analytkonzentration durch Bezug des Meßsignals auf die Eichkurve ermittelt.

Wird ein Nachweisverfahren angewendet, in dem mindestens zwei analytspezifische Rezeptoren notwendig sind, also beispielsweise ein Sandwich-Immunoassay, so wird ein Konjugat aus mindestens zwei Bindungstellen für jeden dieser beiden Rezeptoren, verbunden durch mindestens eine lösliche Trägersubstanz, verwendet. Die beiden Rezeptoren sind unterschiedlich das heißt, sie erkennen unterschiedliche Epitope des Antigens. In diesem Fall enthält das Konjugat mindestens zwei gleiche Bindungsstellen für den ersten Rezeptor und mindestens zwei Bindungsstelen für den zweiten unterschiedlichen Rezeptor. Beispielsweise kann Troponin T auf diese Art nachgewiesen werden.

Die folgenden Beispiele, Abbildungen und das Sequenzprotokoll erläutern die Erfindung weiter.
Fig. 1 zeigt eine bevorzugte Baumstruktur eines erfindungsgemäß geeigneten Konjugats. Dabei sind II und III ebenfalls geeignete Unterstrukturen, T: Spacer, optional; Q: Antikörperbindungsstelle(n)

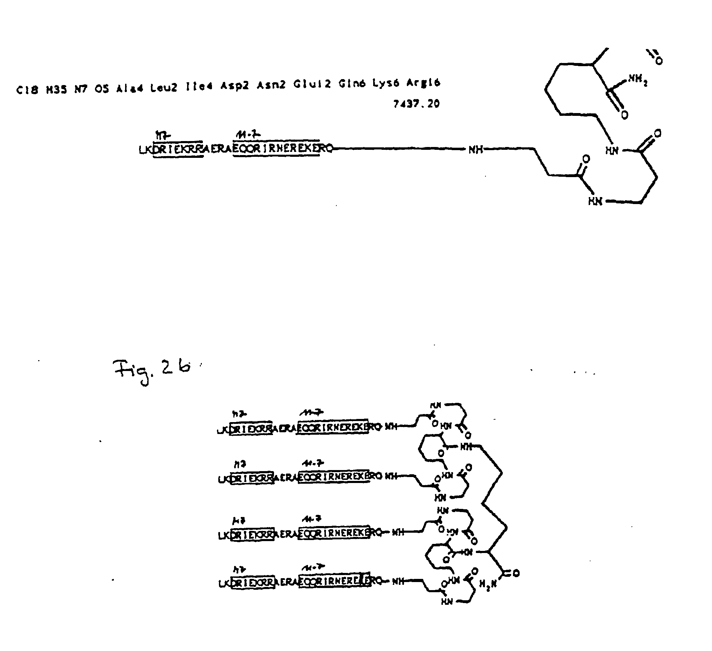

Fig. 2 a und b zeigen die in Beispiel 3 verwendeten synthetischen Kalibratoren.
Fig. 3 zeigt die im Beispiel 8 (Fig. 3a) und 9 (Fig. 3b) synthetisierten Konjugate.
Fig. 4 zeigt die Standardkurven, die mit den synthetischen FM-Konjugaten II und III und einem Fibrin-Standard des Standes der Technik entsprechend Beispiel 10 erhalten wurden.

### Beispiel 1

Herstellung des synthetischen Troponin T-Konjugats Nα, Nε-Bis-(leucyl-lysyl-aspartyl-arginyl-isoleucyl-glutamyl-lysylarginyl-arginyl-alanyl-glutamyl-arginyl-alanyl-glutamyl-glutaminyl-glutaminyl-arginyl-isoleucyl-arginyl-asparagyl-glutamyl-arginyl-glutamyl-lysyl-glutamyl-arginyl-glutaminyl-β-alanyl-β-alanyl)-lysinamid (I)

Das Konjugat (I) wurde mittels Fmoc-(Fluorenylmethoxy-carbonyl-)-Festphasenpeptidsynthese mit einem SMPS 350 Peptidsynthesizer der Fa. ZINSSER Analytic an 15 mg 4-(2',4'-Dimethoxyphenyl-Fmoc-aminomethyl)-phenoxy-Harz SA5030 der Fa. ADVANCED CHEMTECH mit einer Beladung von 0,22 mmol/g hergestellt. Von folgenden N-Fmoc-Aminosäurederivaten wurden nacheinander zweimal je 90 µmol zusammen mit je 90 µmol 1-Hydroxybenzotriazol in 270 µl Dimethylformamid und 1 05 µl einer Dimethylformamidlösung von 90 µmol N,N-Diisopropylcarbodiimid an das aufzubauende festphasengebundene Peptid angekoppelt: Nε-Fmoc-Lysin, β-Alanin, β-Alanin, Glutamin-Trityl), Arginin(-Pentamethylchroman), Glutaminsäure(-tert. (-Butylester), Nε-tert. Butyloxycarbonyl-Lysin, Glutaminsäure-(-tert. Butylester), Arginin(-Pentamethylchroman), Glutamin-säure(-tert. Butylester), Asparagin(-Trityl), Arginin(-Penta-methylchroman), Isoleucin, Arginin(-Pentamethylchroman), Glutamin(-Trityl), Glutamin(-Trityl), Glutaminsäure (tert.Butylester), Alanin, Arginin(-Pentamethylchroman), Glutaminsäure(-tert. Butylester), Alanin, Arginin(-Pentamethylchroman, Arginin(-Pentamethyl-chroman), Nε-tert.Butyloxycarbonyl-Lysin, Glutaminsäure(-tert. Butylester), Isoleucin, Arginin(-Pentamethylchroman), Asparaginsäure(-tert.Butylester), Ne-tert.Butyloxycarbonyl-Lysin, Leucin. Die Kupplungszeiten betragen 40 und 30 Minuten. Die Abspaltungszeit der Fmoc-Schutzgruppe erfolgt nach jeder Doppelkupplung mit 600 µl einer 50 %igen Lösung von Piperidin in Dimethylformamid. Die Abspaltzeit beträgt 20 Minuten. Die Waschschritte werden mit jeweils 700 µl Dimethylformamid achtmal nach jedem der Reaktionsschritte durchgeführt. Die Freisetzung des Peptids erfolgt durch Behandlung des vom Lösungsmittel filtrierten Harzes mit je 750 µl einer Mischung aus 90 % Trifluoressigsäure, 3 % Thioanisol, 3 % Ethandithiol und 3 % Thiokresol innerhalb von 20 Minuten und anschließend 140 Minuten. Das Produkt wird durch Zugabe von 15 ml kaltem Diisopropylether zum vereinigten Filtrat ausgefällt und durch Filtration isoliert. Der Rückstand wird in 3 ml 50 %iger Essigsäure gelöst und lyophilisiert. Der Lyophilisationsvorgang wird zweimal wiederholt. Es werden 10 mg Rohmaterial einer Reinheit von 48 % laut reverse-phase-HPLC erhalten, wovon 5 mg mittels präparativer reverse-phase-HPLC gereinigt werden. Ausbeute: 1 mg (LSIMS : M-H+: 7438.4; Matrix: mNBA, Beschleunigungsspannung: 6 kV).

### Beispiel 2

### Herstellung des synthetischen Troponin T-Konjugats Leucyllysyl-aspartyl-arginyl-isoleucyl-glutamyl-lysyl-arginyl-arglnyl-alanyl-glutamyl-arginyl-alanyl-glutamyl-glutaminyl-glutaminyl-arginyl-isoleucyl-arginyl-aspargyl-glutamyl-arginyl-glutamyl-lysyl-glutamyl-arginyl-glutaminyl-leucyl-lysyl-aspartyl-arginyl-isoleucyl-glutamyl-lysyl-arginyl-arginyl-alanylglutamyl-arginyl-alanyl-glutamyl-glutaminyl-glutaminyl-arginyl-isoleucyl-arginyl-aspargyl-glutamyl-arginyl-glutamyl-lysyl-glutamyl-arginyl-glutamin (II)

Das Konjugat (II) wurde mittels Fmoc-(Fluorenylmethoxy-carbonyl-)-Festphasenpeptidsynthese mit einem SMPS 350 Peptidsynthesizer der Fa. ZINSSER Analytic an 15 mg 4-(2',4'-Dimethoxyphenyl-Fmoc-Aminomethyl)-phen oxy-Harz SA5030 der Fa. Advanced Chemtech mit einer Beladung von 0,22 mmol/g hergestellt. Von folgenden N-Fmoc-Aminosäurederivaten wurden nacheinander zweimal je 90 µmol zusammen mit je 90 µmol 1-Hydroxybenzotriazol in 270 µL DMF (Dimethylformamid) und 105 µL einer DMF-Lösung von 90 µmol N,N-Diisopropylcarbodiimid an das aufzubauendefestpha-sengebundene Peptid angekoppelt: Glutamin(-Trityl), Ariginin (Pentamethylchroman), Glutaminsäure (tert. Butyl-ester), Lysin (tert. Butyloxycarbonyl), Glutaminsäure (tert. Butylester), Arginin (Pentamethylchroman), Glutaminsäure (tert. Butylester), Asparagin (Trityl), Arginin (Pentamethylchroman), Isoleucin, Arginin(Pentamethylchroman), Glutamin (Trityl), Glutamin (Trityl), Glutaminsäure (tert.Butylester), Alanin, Arginin (Pentamethylchroman), Glutaminsäure (tert. Butylester), Alanin, Arginin (Pentamethylchroman), Arginin (Pentamethylchroman), Lysin (tert. Butyloxycarbonyl), Glutaminsäure (tert.Butylester), Isoleucin, Arginin (Pentamethylchroman), Asparaginsäure (tert.Butylester), Lysin (tert. Butyloxycarbonyl), Leucin. Nach Ankopplung des Leucins wird daran nochmals die gleiche Aminosäuresequenz sequentiell ansynthetisiert. Die Kupplungszeiten betragen 40 und 30 Minuten. Die Abspaltzeit der Fmoc-Schutzgruppe erfolgt nach jeder Doppelkupplung mit 600 µL einer 50%igen Lösung von Piperidin in DMF. Die Abspaltzeit beträgt 20 Minuten und wird einmal wiederholt. Die Waschschritte werden mit jeweils 700 µL DMF achtmal nach jedem der Reaktionsschritte durchgeführt. Die Freisetzung des Peptids erfolgt durch Behandlung des vom Lösungsmittel filtrierten Harzes mit je 750 µL einer Mischung aus 90% Trifluoressigsäure, 3% Thioanisol, 3% Ethandithiol und 3% Thiokresol innerhalb von 20 Minuten und anschließend 140 Minuten. Das Produkt wird durch Zugabe von 15 mL kaltem Diisopropylester zum vereinigten Filtrat ausgefällt und durch Filtration isoliert. Der Rückstand wird in 3 mL 50%ige Essigsäure gelöst und lyophilisiert. Der Lyophilisationsvorgang wird zweimal wiederholt. Es werden 17 mg Rohmaterial einer Reinheit von 52% laut reverse-phase-HPLC erhalten, wovon 5 mg mittels präparativer reverse-phase-HPLC gereinigt werden. Es werden 1 mg Reinmaterial (lt. HPLC >99%) erhalten.

### Beispiel 3

### Bestimmung von Troponin T in einem Elektro-Chemilumineszenz-Immunoassay

Ein Biotin-markierter monoklonaler Antikörper (MAK) gegen Troponin T und ein Ruthenium-Tris(bispyridyl)-markierter Antikörperwerden mit Troponin T- und Streptavidin-beladenen magnetischen Polymerpartikeln (Beads) 10 Minuten lang inkubiert. Während der Inkubationszeit bildet sich der Immunkomplex sowie die Bindung des biotinierten MAKs an die Magnetpartikel aus. Die Reaktion wird durch Absaugen des Reaktionsgemisches in die Meßzelle beendet. In der Meßkammer werden die magnetischen Polymerpartikel durch einen Magneten auf der Elektrode fixiert. Das restliche Reaktionsgemisch wird abgesaugt und die Magnetpartikel mit Assay Puffer gewaschen. Anschließend erfolgt die Anregung der Lichtemission durch Anlegen einer elektrischen Spannung.

### Reagenzien:

**Lösung 1:**
   Inkubationspuffer:
      100 mM Natriumphosphat pH 7,0
      0,1 % RSA (Rinderserumalbumin)
      0,1 % Methylisothiazolon
      0,1 % Natriumbenzoat
**Lösung 2:**
   Biotinylierter MAK gegen Troponin T 2 µg/ml in Inkubationspuffer
**Lösung 3:**
   Ruthenium-Tris(bispyridyl)-markierter MAK gegen Troponin T 2 µg/ml in Inkubationspuffer
**Lösung 4:**
   600 µg/ml Magnetbeads, Streptavidin-beschichtet, in:
   50 mM Hepespuffer, 0,1 % RSA, 0,1 % Thesit, 0,1 % Chloracetamid, 0,01 % Methylisothiazolon, pH 7,4
**Lösung 5:**
   Assay Puffer: pH 6,8
   Tris-Propyl-Amin 0,16 M, Di-Kaliumhydrogenphosphat 0,2 M, Polydocanol (Thesit) 0,1 %, Oxaban A 0,1 %

Die zu testenden Konjugate werden in Humanserum oder Inkubationspuffer eingewogen. Das Signal, das man durch Messen dieser Proben in oben beschriebenen Verfahren erhält, wird mit dem Signal ver-glichen, welches mit den Eichlösungen erhalten wird. Daraus wird der Quotient Wiederfindung/Einwaage berechnet.

Je 60 µl Lösung 1 - 4 werden mit 60 µl Probe 10 Minuten lang bei Raumtemperatur inkubiert. Danach wird die Reaktionsmischung in die Meßkammer gesaugt, die Magnetpartikel abgeschieden. Die Meßkammer wird mit Assay Puffer gefüllt und das Meßsignal aufgenommen. Die Konzentration der Proben wird durch Signalvergleich mit Eichlösungen ermittelt.

Die Messung wurde an einem Origen® 1.0-Gerät der Firma Igen, USA durchgeführt.

Die Ergebnisse zeigt Tabelle 1.

**Tabelle 1**

| a) Konjugat I | | | |
|---|---|---|---|
| | Wiederfindung ²⁾ | | |
| | frisch eingewogen | nach 3 Wochen -20°C | nach 3 Wochen 4°C |
| Std a | 218144 ¹⁾ | 95 % | 93 % |
| Std b | 320998 | 88 % | 102 % |
| Std c | 620489 | 94 % | 95 % |
| Std d | 2416923 | 92 % | 90 % |
| Std e | 4275586 | 90 % | 88 % |

| b) Konjugat II | | | |
|---|---|---|---|
| | Wiederfindung ²⁾ | | |
| | frisch eingewogen | nach 3 Wochen -20°C | nach 3 Wochen 4°C |
| Std a | 318143 ¹⁾ | 90 % | 90 % |
| Std b | 4 420798 | 88 % | 100 % |
| Std c | 720789 | 90 % | 92 % |
| Std d | 2616522 | 93 % | 85 % |
| Std e | 4576789 | 88 % | 83 % |

| c) human-herz-Troponin T ³⁾ | | | |
|---|---|---|---|
| | Wiederfindung ²⁾ | | |
| | frisch eingewogen | nach 3 Wochen -20°C | nach 3 Wochen 4°C |
| Std a | 241233 ¹⁾ | 95 % | 60 % |
| Std b | 467345 | 98 % | 58 % |
| Std c | 814325 | 93 % | 55 % |
| Std d | 2456342 | 88 % | 63 % |
| Std e | 4675432 | 89 % | 59 % |

| | | | |
|---|---|---|---|
| ¹⁾ Lichtemissionseinheiten | | | |
| ²⁾ Wiederfindung in % des Meßergebnisses, das unmittelbar nach Herstellung der Lösungen erhalten wird. Lagerung bei 35°C. | | | |
| ³⁾ Std: Troponinstandard (bisher gebräuchlicher Standard aus Enzymuntest TnT, Boehringer Mannheim GmbH) | | | |

### Beispiel 4

### Herstellung des Troponin T-Konjugates (III)

200 mg Rinderserumalbumin (2,9 µMol) werden in 6 ml 0,1 M Kaliumphosphatpuffer pH 8,0 gelöst. 44,6 mg Maleinimidohexansäure-N-hydroxysuccinimidester (145 µMol) werden in 0,5 ml Dioxan gelöst und zur obigen Lösung getropft. Man läßt 17 Stunden bei Raumtemperatur (RT) rühren.

Die obige Lösung wird über eine mit Aca 202® (Pharmacia) gefüllte Säule (3 cm x 40 cm), die mit 0,1 M Kaliumphosphatpuffer pH 6,0 equilibriert wurde, filtriert (Detektion λ = 226 nm). Der erste Peak wird gesammelt und dessen Proteingehalt nach der Methode von Pierce (BCA-Methode) ermittelt. Die erhaltene Beladung an Maleinimidohexanoyl-Gruppen wird nach der Methode von Ellman ermittelt. Sie beträgt 17,5 Maleinimidohexanoyl-Gruppen pro Molekül Rinderserumalbumin.

10 mg Cystelnyl-β-alanyl-β-alanyl-alanyl-glutanyl-glutaminyl-glutaminyl-arginyl-soeucyl-arginyl-asparaginylglutamyl-arginyl-glutamyl-lysyl-glutamyl-arginyl-amid (4,79 µmol) und 8,2 mg Cysteinyl- -aminocaproyl-seryl-leucyllysyl-aspartyl-arginyl-isoleucyl-glutamyl-lysyl-arginyl-arginyl-alanyl-glutamyl-amid (4,79 µmol) werden in 1 ml 0,1 M Kaliumphosphatpuffer pH 6,0 gelöst. Die Lösung wird mit Argon gesättigt. Man gibt 2,13 ml einer Lösung von maleinmido-hexanoyl-aktiviertem Rinderserumalbimin (Beladung 17,5 : 1) mit einer Konzentration von 9,46 mg/ml in 0,1 M Kaliumphosphatpuffer pH 6,0 zu obiger Lösung. Man sättigt erneut mit Argon und rührt unter Sauerstoffausschluß bei RT 17 Stunden. Es wird 4 ml 0,05 M Bernsteinsäurepuffer pH 4,0 zugegeben und 4 mal gegen 1 1 1 %iger Essigsäure dialysiert. Der unlösliche Niederschlag wird abzentrifugiert.

Die obige Lösung wird über eine mit Aca 202 (Pharmacia) gefüllte Säule (3 cm x 28 cm), die mit 0,05 M Bernsteinsäurepuffer pH 4,0 equilibriert wurde, filtiert (Detektion λ = 226 nm). Der erste Peak wird gesammelt und dessen Proteingehalt nach der Methode von Pierce (BCA-Methode) ermittelt. Es werden 19,8 mg Proteinkonjugat mit 35 ml Lösung gefunden. Nach der Methode von Ellman wird keine Maleinimido-Aktivität mehr nachgewiesen.

### Beispiel 5

### Herstellung des Troponin T Konjugates (IV)

200 mg Rinderserumalbumin (2,9 µMol) werden in 6 ml 0,1 M Kaliumphosphatpuffer pH 8,0 gelöst. 44,6 mg Maleinimidohexansäure-N-hydroxysuccinimidester (145 µMol) werden in 0,5 ml Dioxan gelöst und obiger Lösung zugesetzt. Man läßt 17 Stunden bei Raumtemperatur rühren.

Die obige Lösung wird über eine mit Aca 202 (Pharmacia) gefüllte Säule (3 cm x 40 cm), die mit 0,1 M Kallumphosphatpuffer pH 6,0 equilibriert wurde, filtriert (Detektion λ = 226 nm). Der erste Peakwird gesammelt und dessen Proteingehalt nach der Methode von Pierce (BCA-Methode) ermittelt. Die erhaltene Beladung an Maleinimidohexanoyl-Gruppen wird nach der Methode von Ellman mit 18,0 pro Molekül Rinderserumalbumin ermittelt.

35 mg Cysteinyl- -aminocaproyl-leucyl-lysyl-aspartyl-arginyl-isoleucyl-glutamyl-lysyl-arginyl-arginyl-alanylglutamyl-arginyl-alanyl-glutamyl-glutamainyl-glutaminyl-arginyl-isoleuyl-arginyl-aspargyl-glutamyl-arginyl-glutamyllysyl-glutamyl-arginyl-glutamyl-amid (9,6 µMol) werden in 3 ml 0,1 M Kaliumphosphatpuffer pH 6,0 und die Lösung mit Argon gesättigt. Von der maleinimidohexanoly-aktivierten Rinderserumalbuminlösung (c = 9,2 mg/ml) werden 2,35 ml zur Peptid-Lösung zugegeben und diese Gesamtlösung nochmals mit Argon gesättigt. Die Lösung wird 17 Stunden bei RT gerührt. Nach Zugabe von 0,5 ml 50 %iger Essigsäure wird über eine G4-Glasfilterfritte filtriert. Das Eluat wird über eine mit Aca 202 (Pharmacia) gefüllte Säule (3 cm x 40 cm), die mit 0,1 M Kaliumphosphatpuffer pH 6,0 equilibriert wude, filtriert (Detektion = 226 nm). Der erste Peak wird gesammelt und dessen Proteingehalt nach der methode von Pierce (BCA-Methode) ermittelt. Es werden 25 mg Proteinkonjugat in 25,5 ml Eluat gefunden. Nach der Methode von Ellman wird keine Maleinimido-Aktivität mehr nachgewiesen.

### Beispiel 6

### Stabilitäten des synthetischen Troponin T-Kalibrators (I)

Die Stabilität des Troponin T-Konjugats (I) entsprechend Beispiel 1 wurde mit der Stabilität eines Kalibrators aus natiem Antigen verglichen. Als natives Antigen wurde Troponin T aus Rinderherz (bcTnT) und Human-Herz (hcTNT) verwendet.

Die Kalibratoren wurden bei Temperaturen von -80°C, 4°C und 25°C bis zu drei Wochen lang gelagert. Wöchentlich wurde das mit diesem Kalibrator erhaltene Meßsignal ermittelt und mit dem Ausgangssignal verglichen. Die Testdurchführung entspricht Beispiel 3.

Bei der Aufbewahrung bei -80°C ergaben sich keine Unterschiede. Alle Kalibratoren zeigten nach 3 Wochen noch eine 100%ige Stabilität. Auch bei der Aufbewahrung bei 4°C ergaben sich keine signifikanten Unterschiede. Lediglich bei bcTnT fiel das Meßsignal nach 3 Wochen auf 92 % des Ausgangssignals ab.

Bei der Aufbewahrung bei 25°C zeigte sich die überlegene Stabilität des erfindungsgemäßen Kalibrators. Wähend das Meßsignal bei 25°C nach 3 Wochen bei hcTnT und bcTnT auf 24 beziehungsweise 5 % des Ausgangssignals abfiel, beträgt das Signal des synthetischen Kalibrators noch 50 % des Ausgangswertes.

### Beispiel 7

### Herstellung des synthetischen FM-Konjugats (I) für den Test auf lösliches Fibrin

Das FM-Konjugat (I) für den Test auf lösliches Fibrin wurde mittels Fmoc-(Fluorenylmethoxycarbonyl-)-Festphasenpeptid-synthese mit einem SMPS 350 Peptidsynthesizer der Fa. ZINSSER Analytic an 15 mg 4-(2',4'-Dimethoxyphenyl-Fmoc-aminomethyl)-phenoxy-Harz SA 5030 der Fa. ADVANCED CHEMTECH mit einer Beladung von 0,22 mMol/g hergestellt. Von folgenden N-Fmoc-Aminosäure-Derivaten wurden nacheinander zweimal je 90 µMol zusammen mit je 90 µMol 1-Hydroxybenzotriazol in 270 µl Dimethylformamid und 105 µl einer Dimethylformamidlösung von 90 µMol N,N-Diisopropylcarbodiimid an das aufzubauende festphasengebundene Peptid angekoppelt: Ne-Fmoc-Lysin, β-Alanin, β-Alanin, Arginin(-Pentamethylchroman), Glutaminsäure(-tert. Butylester), Valin, Valin, Argin(-Pentamethylchroman), Prolin und Glycin. Die Kupplungszeiten betragen 40 und 30 Minuten. Die Abspaltungszeit der Fmoc-Schutzgruppe erfolgt nach jeder Doppelkupplung mit 600 µl einer 50 %igen Lösung von Piperidin in Dimethylformamid. Die Waschschritte werden mit jeweils 700 µl Dimethylformamid 8 mal nach jedem der Reaktionsschritte durchgeführt. Die Freisetzung des Peptids erfolgt durch Behandlung des vom Lösungsmittel filtrierten Harzes mit je 750 µl einer Mischung 90 % Trifluoressigsäure, 3 % Thioanisol,, 3 % Ethandithiol und 3 % Thiochresol innerhalb von 20 Minuten und anschließend 140 Minuten. Das Produkt wird durch Zugabe von 15 ml kaltem Diisopropylether zum vereinigten Filtrat ausgefällt und durch Filtration isoliert. Der Rückstand wird in 3 ml 50 %iger Essigsäure gelöst und lyophilisiert. Der Lyophilisationsvorgang wird zweimal wiederholt. Es werden 7 mg Rohmaterial einer Reinheit vo 53 % laut revers-phase-HPLC erhalten, wovon 5 mg mittels präparativer reverse-phase-HPLC gereinigt werden. Ausbeute: 1,25 mg (LSIMS: M-H⁺ : 2300,5; Matrix: mNBA, Beschleunigungsspannung: 6 kV).

Auf diese Weise entsteht ein Konjugat aus zwei Peptiden entsprechend der in SEQ ID NO. 11 dargestellen Sequenz, die jeweils über einen Spacer aus jeweils 2 Molekülen β-Alanin an Lysin gebunden sind. auf lösliches Fibrin

Das FM-Konjugat (II) für den Test auf lösliches Fibrin wurde mittels Fmoc-(Fluorenylmethoxycarbonyl-)-Festphasenpeptidsynthese mit einem SMPS 350 Peptidsynthesizer der Fa. ZINSSER Analytic an 15 mg 4-(2',4'-Dimethoxyphenyl-Fmoc-aminomethyl)-phenoxy-Harz SA 5030 der Fa. ADVANCED CHEMTECH mit einer Beladung von 0,22 mMol/g hergestellt. Von folgenden N-Fmoc-Aminosäure-Derivaten wurden nacheinander zweimal je 90 µMol zusammen mit je 90 µMol 1-Hydroxybenzotriazol in 270 µl Dimethylformamid und 105 µl einer Dimethylformamidlösung von 90 µMol N,N-Diisopropylcarbodiimid an das aufzubauende festphasengebundene Peptid angekoppelt: N -Fmoc-Lysin, Nε-Fmoc-Lysin, β-Alanin, β-Alanin, Arginin(-Pentamethylchroman), Glutaminsäure(-tert. Butylester), Valin, Valin, Argin(-Pentamethylchroman), Prolin und Glycin. Die Kupplungszeiten betragen 40 und 30 Minuten. Die Abspaltungszeit der Fmoc-Schutzgruppe erfolgt nach jeder Doppelkupplung mit 600 µl einer 50 %igen Lösung von Piperidin in Dimethylformamid. Die Waschschritte werden mit jeweils 700 µl Dimethylformamid 8 mal nach jedem der Reaktionsschritte durchgeführt. Die Freisetzung des Peptids erfolgt durch Behandlung des vom Lösungsmittel filtrierten Harzes mit je 750 µl einer Mischung 90 % Trifluoressigsäure, 3 % Thioanisol, 3 % Ethandithiol und 3 % Thiochresol innerhalb von 20 Minuten und anschließend 140 Minuten. Das Produkt wird durch Zugabe von 15 ml kaltem Diisopropylether zum vereinigten Filtrat ausgefällt und durch Filtration isoliert. Der Rückstand wird in 3 ml 50 %iger Essigsäure gelöst und lyophilisiert. Der Lyophilisationsvorgang wird zweimal wiederholt. Es werden 15 mg Rohmaterial einer Reinheitvo 48 % laut revers-phase-HPLC erhalten, wovon 5 mg mittels präparativer reverse-phase-HPLC gereinigt werden. Ausbeute: 1,02 mg (LSIMS: M-H⁺ : 4854,3; Matrix: mNBA, Beschleunigungsspannung: 6 kV).

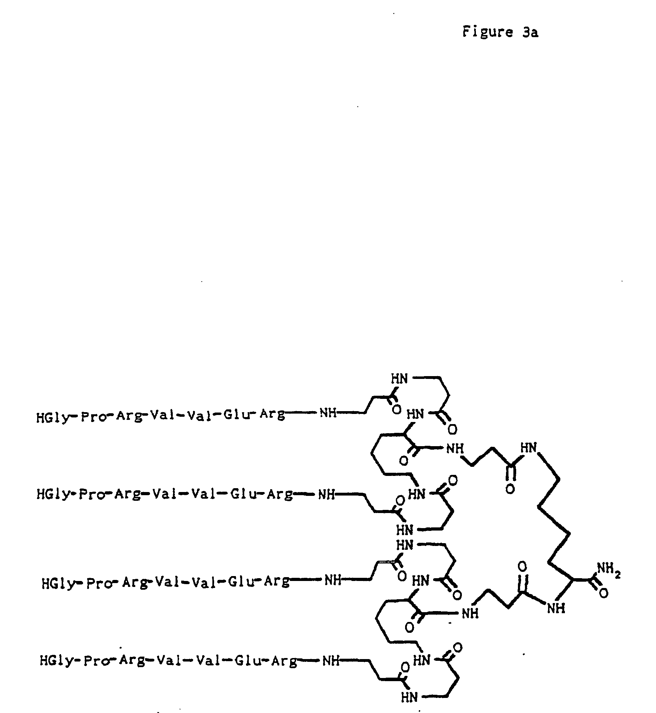

Auf diese Weise entsteht ein Konjugat aus vier Peptiden entsprechend der in SEQ ID NO. 11 dargestellen Sequenz, die jeweils über einen Spacer aus 2 Molekülen an einen Träger aus 3 Lysinresten gebunden sind (Figur 3a).

### Beispiel 9

### Herstellung des synthetischen FM-Konjugats (III) für den Test auf lösliches Fibrin

Das FM-Konjugat (III) für den Test auf lösliches Fibrin wurde mittels Fmoc-(Fluorenylmethoxycarbonyl-)-Festphasenpeptidsynthese mit einem SMPS 350 Peptidsynthesizer der Fa. ZINSSER Analytic an 15 mg 4-(2',4'-Dimethoxyphenyl-Fmoc-aminomethyl)-phenoxy-Harz SA 5030 der Fa. ADVANCED CHEMTECH mit einer Beladung von 0,22 mMol/g hergestellt. Von folgenden N-Fmoc-Aminosäure-Derivaten wurden nacheinander zweimal je 90 µMol zusammen mit je 90 µmol 1-Hydroxybenzotriazol in 270 µl Dimethylformamid und 105 µl einer Dimethylformamidlösung von 90 µMol N,N-Diisopropylcarbodiimid an das aufzubauende festphasengebundene Peptid angekoppelt: Nε-Fmoc-Lysin, Nε-Fmoc-Lysin, Ne- Fmoc-Lysin, β-Alanin, β-Alanin, Arginin(-Pentamethylchroman), Glutaminsäure(-tert. Butylester), Valin, Valin, Argin(-Pentamethylchroman), Prolin und Glycin. Die Kupplungszeiten betragen 40 und 30 Minuten. Die Abspaltungszeit der Fmoc-Schutzgruppe erfolgt nach jeder Doppelkupplung mit 600 µl einer 50 %igen Lösung von Piperidin in Dimethylformamid. Die Waschschritte werden mit jeweils 700 µl Dimethylformamid 8 mal nach jedem der Reaktionsschritte durchgeführt. Die Freisetzung des Peptids erfolgt durch Behandlung des vom Lösungsmittel filtrierten Harzes mit je 750 µl einer Mischung 90 % Trifluoressigsäure, 3 % Thioanisol, 3 % Ethandithiol und 3 % Thiochresol innerhalb von 20 Minuten und anschließend 140 Minuten. Das Produkt wird durch Zugabe von 15 ml kaltem Diisopropylether zum vereinigten Filtrat ausgefällt und durch Filtration isoliert. Der Rückstand wird in 3 ml 50 %iger Essigsäure gelöst und lyophilisiert. Der Lyophilisationsvorgang wird zweimal wiederholt. Es werden 23 mg Rohmaterial einer Reinheit von 42 % laut revers-phase-HPLC erhalten, wovon 5 mg mittels präparativer reverse-phase-HPLC gereinigt werden. Ausbeute: 0,95 mg.

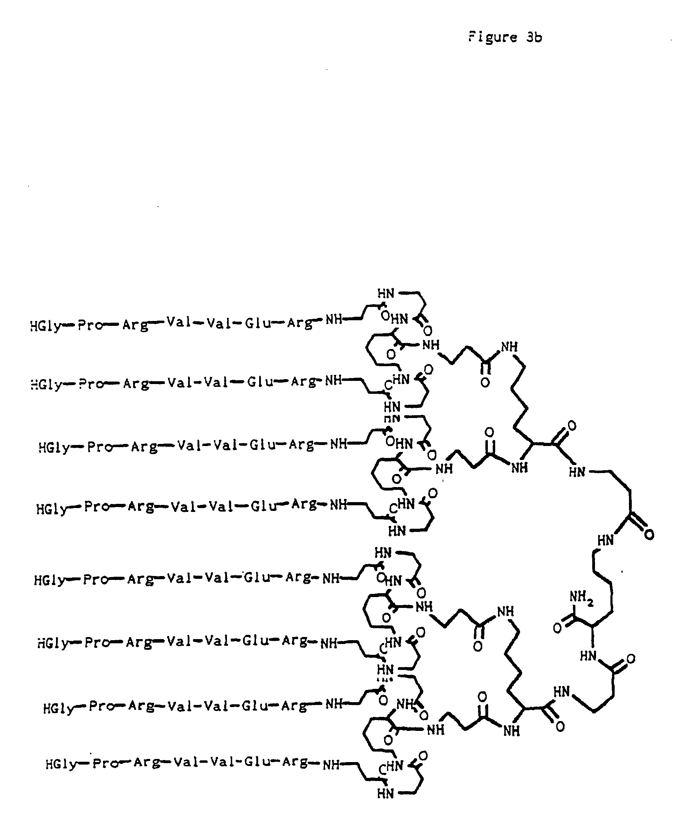

Auf diese Weise entsteht ein Konjugat aus acht Peptiden entsprechend der in SEQ ID NO. 11 dargestellen Sequenz, die jeweils über einen Spacer aus 2 β-Alanin-Molekülen an einen Träger aus 7 Lysin-Resten gebunden sind (Figur 3b).

### Beispiel 10

### Vergleich eines Fibrin Standards mit den synthetischen FM-Konjugaten I, II und III in einem Test auf lösliches Fibrin

Die Herstellung von Fibrin, des Fibrin-Standards und der Fibrin-Abbauprodukte sowie die Behandlung der Probe erfolgte nach Lill et al., Blood Coagulation and Fibrinlysis 4 (1993) 97 - 102.

### Testprinzip

Es wurde ein Zwei-Schritt Sandwich-Assay unter Verwendung von Streptavidin-beschichteten Röhrchen als Festphase durchgeführt. Der gleiche Fibrin-spezifische monoklonale Antikörper wurde sowohl als biotinylierter Fang-MAK und als Peroxidasemarkierter MAK genutzt.

Der Nachweis wurde bei Raumtemperatur an einem ES 33-Gerät durchgeführt.

20 µl Plasma wurden mit 60 µl Inkubationspuffer (5,33 M KSCN, 0,025 M Natriumphosphat, pH 7,3) 30 Minuten in Streptavidin-beschichteten Polystyrolröhrchen (Boehringer Mannheim GmbH) inkubiert. 1000 µl biotinylierte Antikörper-Lösung (1,3 µg/ml biotinylierter Antikörper, 0,1 M Kaliumphosphat, 5,9 mg/ml Rinderserumalbumin, 0,5 mg/ml Tween 20®, pH 7,0) wurden zugesetzt und 30 Minuten inkubiert. Der Antikörper wurde durch Immunisierung von Balb/c Mäusen mit synthetischen N-terminalen Heptapeptid Gly-Pro-Arg-Val-Val-Glu-Arg aus der α-Gruppe von Fibrin erhalten. Das Peptid wurde mittels Maleinimidobenzoyl-N-hydroxy-succinimidester auf Keyhole limpet haemocyanin gekoppelt. Die monoklonalen Antikörper wurden nach Köhler und Milstein, Nature 256 (1975), 495 - 497 erhalten. Anschließend wurde das Röhrchen mit 4,3 mM NaCl-Lösung gewaschen. Danach wurden 1000 µl Peroxidase-markierte Antikörper-Lösung (0,14 U/ml Peroxidase-Konjugat, 0,035 M Kaliumphosphat, 0,154 M NaCl, 10 mg/ml Polyethylenglykol 40000, 2 mg/ml Rinderserumalbumin, 0,5 mg/ml Tween20®, pH 7,4) zugegeben und 30 Minuten inkubiert. Danach wurde die Lösung aus dem Röhrchen entfernt und dieses mit 4,3 mM NaCl-Lösung gewaschen.

1000 µl ABTS® -Lösung (Boehringer Mannheim GmbH) wurde zugesetzt und 30 Minuten inkubiert. Die Adsorption wurde danach bei 422 nm Wellenlänge gemessen und die Fibrin-Konzentration durch Vergleich mit der Standardkurve ermittelt.

### Standardkurven

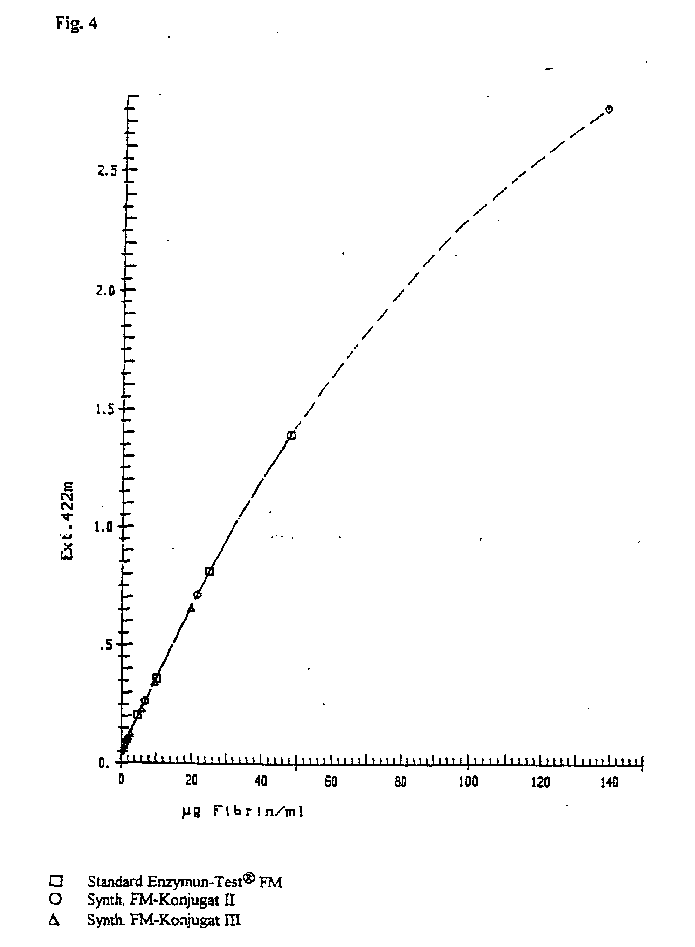

Figur 4 zeigt Standardkurven. Diese wurden im Bereich von 0 bis 50 µg/ml Fibrin ermittelt. Als Standards wurden der Fibrin-Standard des Enzymun-Test FM (Boehringer Mannheim GmbH) und die synthetischen FM-Konjugate II und III verwendet. Lyophilisierte Plasma-Proben, die mit den FM-Konjugaten aufgestockt wurden, dienten als Standards.

Die Ergebnisse sind in der Figur 4 dargestellt. Es zeigt sich eine sehr hohe Korrelation zwischen den erfindungsgemäßen Konjugaten und dem Standard des Standes der Technik.

## Patentansprüche

1. Verwendung eines flüssig stabilen Kalibrators in einem Test zum Nachweis eines Analyten, **dadurch gekennzeichnet, dass** besagter Kalibrator ein Konjugat bestehend aus jeweils mindestens zwei gleichen Bindestellen, die mit zwei unterschiedlichen Rezeptoren, die spezifisch mit der analytspezifischen Binderegion besagter Rezeptoren, die zum Nachweis im Test eingesetzt werden, binden, wobei die Bindungstellen durch mindestens eine lösliche Trägersubstanz verbunden sind und das Konjugat das Produkt einer synthetischen oder rekombinanten Herstellung ist und die eingesetzte Menge genau bekannt ist.

2. Verwendung eines Kalibrators nach Anspruch 1, **dadurch gekennzeichnet, dass** als Bindungsstelle ein Peptid, ein Peptidderivat oder ein Peptidmimetikum verwendet wird.

3. Verwendung eines Kalibrators nach Anspruch 2, **dadurch gekennzeichnet, dass** das Peptid eine Länge von mindestens vier Aminosäuren beziehungsweise das Peptidderivat oder das Peptidmimetikum eine Länge analog mindestens vier Aminosäuren aufweist.

4. Verwendung eines Kalibrators nach Anspruch 3, **dadurch gekennzeichnet, dass** das Peptid eine Länge von 4 - 20 Aminosäuren beziehungsweise das Peptidderivat oder das Peptidmimetikum eine Länge analog 4 - 20 Aminosäuren aufweist.

5. Verwendung eines Kalibrators nach einem der Ansprüche 1 - 4, **dadurch gekennzeichnet, dass** 2-50 Bindungsstellen in dem Konjugat enthalten sind.

6. Verwendung eines Kalibrators nach einem der Ansprüche 1 - 5, **dadurch gekennzeichnet, dass** die Bindungsstellen spezifisch mit einem analytspezifischen Paratop eines Antikörpers oder eines Antikörperfragments, der als Rezeptor im Test eingesetzt wird, binden.

7. Verwendung eines Kalibrators nach einem der Ansprüche 1-6, **dadurch gekennzeichnet, dass** die wässrige Lösung zusätzlich weitere Hilfsstoffe wie Puffer und Stabilisatoren enthält.

8. Verwendung eines Kalibrators nach Anspruch 1, **dadurch gekennzeichnet, dass** als Bindungsstellen mindestens zwei verschiedene Peptide ausgewählt aus einer Gruppe von Peptiden mit der Aminosäuresequenz der Sequenzprotokolle SEQ ID NO. 1-10 enthalten sind, in einem Test zum Nachweis von Troponin T.

9. Verfahren zum Nachweis eines Analyten in einer Probe durch Bindung des Analyten an mindestens zwei spezifische Rezeptoren und Nachweis des gebildeten Komplexes als Maß für den Gehalt des Analyten in der Probe, **dadurch gekennzeichnet, dass**
a) in einem ersten Schritt eine bekannte Menge eines Konjugates aus jeweils mindestens zwei gleichen Bindungsstellen, die mit zwei unterschiedlichen Rezeptoren, die spezifisch mit der analytspezifischen Binderegion besagter Rezeptorens, die zum Nachweis im Test eingesetzt werden, binden, wobei die Bindungsstellen durch mindestens eine lösliche Trägersubstanz verbunden sind und das Konjugat das Produkt einer synthetischen oder rekombinanten Herstellung ist, anstelle der Probe mit den analytspezifischen Rezeptoren gebunden wird.
b) der gebildete Komplex zwischen Konjugat und Rezeptor bestimmt wird,
c) das Meßergebnis aus Schritt b) zur Erstellung einer Eichkurve verwendet wird,
d) der Nachweis des Analyten in der Probe durchgeführt wird und die Analytkonzentration durch Bezug des Meßsignals auf die aus c) erhaltene Eichkurve ermittelt wird.

10. Flüssig stabiler synthetischer Kalibrator zur Verwendung in einem Test zum Nachweis eines Analyten, **dadurch gekennzeichnet, dass** er ein Konjugat aus mindestens zwei gleichen Bindungsstellen, welche spezifisch an einen ersten Rezeptor binden, und mindestens zwei gleichen Bindungsstellen, welche spezifisch an einen zweiten Rezeptor binden, wobei die Bindungsstellen durch mindestens eine lösliche Trägersubstanz verbunden sind und das Konjugat das Produkt einer synthetischen oder rekombinanten Herstellung ist, enthält, wobei die beiden Rezeptoren nicht oder nicht signifikant um die gleiche Bindungsstelle kompetieren.

## Claims

1. Use of a liquid-stable calibrator in a test for the detection of an analyte, **characterized in that** said calibrator is a conjugate composed in each case of at least two identical binding sites which bind to two different receptors that bind specifically to the analyte-specific binding region of the said receptors which are used for detection in the test, wherein the binding sites are linked by at least one soluble carrier substance and the conjugate is a product of a synthetic or recombinant production and the amount used is exactly known.

2. Use of a calibrator as claimed in claim 1, **characterized in that** a peptide, a peptide derivative or a peptide mimetic is used as the binding site.

3. Use of a calibrator as claimed in claim 2, **characterized in that** the peptide has a length of at least four amino acids or the peptide derivative or the peptide mimetic has a length corresponding to at least four amino acids.

4. Use of a calibrator as claimed in claim 3, **characterized in that** the peptide has a length of 4 - 20 amino acids or the peptide derivative or the peptide mimetic has a length corresponding to 4 - 20 amino acids.

5. Use of a calibrator as claimed in one of the claims 1 - 4, **characterized in that** the conjugate contains 2 - 50 binding sites.

6. Use of a calibrator as claimed in one of the claims 1 - 5, **characterized in that** the binding sites bind specifically to an analyte-specific paratope of an antibody or of an antibody fragment that is used as a receptor in the test.

7. Use of a calibrator as claimed in one of the claims 1 - 6, **characterized in that** the aqueous solution additionally contains other auxiliary substances such as buffers and stabilizers.

8. Use of a calibrator as claimed in claim 1, **characterized in that** it comprises at least two different peptides selected from a group of peptides having the amino acid sequence of the sequence protocols SEQ ID NO. 1 - 10 as binding sites in a test for the detection of troponin T.

9. Method for the detection of an analyte in a sample by binding the analyte to at least two specific receptors and detecting the complex that is formed as a measure of the content of analyte in the sample, **characterized in that**
a) in a first step a known amount of a conjugate comprising in each case at least two identical binding sites which bind to two different receptors that specifically bind to the analyte-specific binding region of the said receptors which are used for detection in the test wherein the binding sites are linked by at least one soluble carrier substance and the conjugate which is a product of a synthetic or recombinant production, is bound to the analyte-specific receptors instead of the sample,
b) the complex formed between the conjugate and receptor is determined,
c) the result of the measurement from step b) is used to plot a calibration curve,
d) the analyte is detected in the sample and the analyte concentration is determined by comparing the measured signal with the calibration curve obtained from c).

10. Liquid-stable synthetic calibrator for use in a test for detecting an analyte, **characterized in that** it contains a conjugate comprising at least two identical binding sites which bind specifically to a first receptor and at least two identical binding sites which bind specifically to a second receptor wherein the binding sites are linked by at least one soluble carrier substance and the conjugate is a product of a synthetic or recombinant production wherein the two receptors do not compete or do not significantly compete for the same binding site.

## Revendications

1. Utilisation d'un agent d'étalonnage stable liquide dans un essai pour le décèlement d'un analyte, **caractérisée en ce que** ledit agent d'étalonnage est un conjugué constitué par à chaque fois au moins deux sites de liaison identiques qui se lient spécifiquement à la zone de liaison spécifique à l'analyte de deux récepteurs différents, qui sont utilisés pour le décèlement dans le test, les sites de liaison étant reliés par au moins une substance support soluble et le conjugué est le produit d'une préparation synthétique ou recombinante et la quantité mise en oeuvre est connue avec précision.

2. Utilisation d'un agent d'étalonnage selon la revendication 1, **caractérisée en ce qu'**on utilise, à titre de site de liaison, un peptide, un dérivé de peptide ou une substance mimétique d'un peptide.

3. Utilisation d'un agent d'étalonnage selon la revendication 2, **caractérisée en ce que** le peptide présente une longueur d'au moins quatre acides aminés, respectivement le dérivé de peptide ou la substance mimétique d'un peptide présente une longueur analogue à au moins quatre acides aminés.

4. Utilisation d'un agent d'étalonnage selon la revendication 3, **caractérisée en ce que** le peptide présente une longueur de 4 - 20 acides aminés, respectivement le dérivé de peptide ou la substance mimétique d'un peptide présente une longueur analogue à 4 - 20 acides aminés.

5. Utilisation d'un agent d'étalonnage selon l'une quelconque des revendications 1 à 4, **caractérisée en ce que** 2 à 50 sites de liaison sont contenus dans le conjugué.

6. Utilisation d'un agent d'étalonnage selon l'une quelconque des revendications 1 à 5, **caractérisée en ce que** les sites de liaison se lient de manière spécifique à un paratope spécifique à l'analyte d'un anticorps ou d'un fragment d'anticorps qui est mis en oeuvre à titre de récepteur dans l'essai.

7. Utilisation d'un agent d'étalonnage selon l'une quelconque des revendications 1 à 6, **caractérisée en ce que** la solution aqueuse contient en outre des adjuvants supplémentaires tels que des tampons et des stabilisateurs.

8. Utilisation d'un agent d'étalonnage selon la revendication 1, **caractérisée en ce qu'**au moins deux peptides différents choisis dans un groupe de peptides présentant la séquence d'acides aminés du protocole de séquence SEQ ID N° 1-10, sont contenus à titre de sites de liaison dans un essai pour déceler la présence de troponine T.

9. Procédé pour déceler un analyte dans un échantillon par liaison de l'analyte à au moins deux récepteurs spécifiques et par décèlement du complexe obtenu comme mesure pour la teneur de l'analyte dans l'échantillon, **caractérisé en ce que**
a) dans une première étape, on lie une quantité connue d'un conjugué constitué par à chaque fois au moins deux sites de liaison identiques qui se lient spécifiquement à la zone de liaison spécifique à l'analyte de deux récepteurs différents, qui sont utilisés pour le décèlement dans le test, les sites de liaison étant reliés par au moins une substance support soluble et le conjugué est le produit d'une préparation synthétique ou recombinante, à la place de l'échantillon avec les récepteurs spécifiques à l'analyte,
b) on détermine le complexe formé entre le conjugué et le récepteur,
c) on utilise le résultat de mesure de l'étape b) pour construire une courbe d'étalonnage,
d) on procède au décèlement de l'analyte dans l'échantillon et on détermine la concentration en analyte par référence au signal de mesure sur la courbe d'étalonnage obtenue à partir de c).

10. Agent d'étalonnage synthétique stable liquide à utiliser dans un essai pour le décèlement d'un analyte, **caractérisé en ce qu'**il contient un conjugué constitué par au moins deux sites de liaison identiques, qui se lient de manière spécifique à un premier récepteur, et au moins deux sites de liaison identiques, qui se lient de manière spécifique à un deuxième récepteur, les deux sites de liaison étant reliés par au moins une substance support soluble et le conjugué étant le produit d'une préparation synthétique ou recombinante, les deux récepteurs n'entrant pas ou n'entrant essentiellement pas en concurrence réciproque pour le même site de liaison.
